# EUROPÄISCHE PATENTSCHRIFT

(11) **EP 2 665 498 B1**
(45) Veröffentlichungstag und Bekanntmachung des Hinweises auf die Patenterteilung: **03.05.2017**
(21) Anmeldenummer: 12700771.4
(22) Anmeldetag: 12.01.2012
(51) Int. Cl.: A61M 1/00

(54) **DRAINAGEPUMPEINHEIT**
DRAINAGE PUMP UNIT
ENSEMBLE POMPE DE DRAINAGE

(30) Priorität: 17.01.2011 CH 78112011
(43) Veröffentlichungstag der Anmeldung: 27.11.2013
(73) Patentinhaber: Medela Holding AG, 6340 Baar (CH)
(72) Erfinder: GIEZENDANNER, Charles, CH-6443 Morschach (CH); MELZER, Martin, A-6330 Cham (CH)
(74) Vertreter: Clerc, Natalia
(86) Internationale Anmeldenummer: PCT/CH2012/000007
(87) Internationale Veröffentlichungsnummer: WO 2012/097462

(56) Entgegenhaltungen:
- EP-A1- 0 853 950
- WO-A1-96/33393
- WO-A1-2006/021295
- WO-A1-2009/098077
- US-A- 5 747 689

## Beschreibung

### TECHNISCHES GEBIET

Die vorliegende Erfindung betrifft eine Drainagepumpeinheit gemäss Oberbegriff des Patentanspruchs 1 sowie einen Füllstandsensor gemäss Oberbegriff des Patentanspruchs 15.

### STAND DER TECHNIK

Zum Absaugen von Körperflüssigkeiten bzw. -fluiden im medizinischen Bereich werden Drainagepumpsysteme eingesetzt. Sie werden beispielsweise bei bzw. nach chirurgischen Eingriffen, in der Wunddrainage, der Thoraxdrainage oder beim Absaugen von Körperfetten verwendet. Diese Drainagepumpsysteme weisen üblicherweise eine Vakuumpumpe, einen oder mehrere Fluidsammelbehälter und eine Drainageschlauchverbindung zwischen Patient und Fluidsammelbehälter auf. Der Fluidsammelbehälter kann am Gehäuse der Drainagepumpe lösbar befestigt sein, oder er ist mit der Pumpe über einen externen Vakuumschlauch verbunden.

Indem mittels der Saug- bzw. Vakuumpumpe ein Unterdruck im Fluidsammelbehälter erzeugt wird, lässt sich das Fluid oder das Sekret von einer Kavität des Patienten über den Drainage- oder Sekretschlauch in den Sammelbehälter saugen und dort sammeln. Am pumpenseitigen Ausgang des Sammelbehälters angeordnete Filter schützen die Saugpumpe vor einer allfälligen Verunreinigung durch das abgesaugte Fluid. Derartige Drainagepumpsysteme sind beispielsweise in WO 2007/128156 und in WO 2008/141471 offenbart. Die dort beschriebenen Drainagepumpsysteme sind tragbar ausgebildet.

Zur Überwachung des Drainageverlaufs und des Füllstandes des Fluidsammelbehälters sind kapazitive Füllstandsensoren geeignet.

Füllstandsensoren sind im Stand der Technik bekannt. So offenbart US 4 099 167 einen kapazitiven Füllstandsensor zur Bestimmung eines Füllstandes eines Flüssigkeitsbehälters. Der Füllstandsensor weist in einer Ausführungsform eine bandförmige erste Elektrode und mehrere beabstandet dazu angeordnete zweite Elektroden auf. Die zweiten Elektroden sind beabstandet zueinander in vertikaler Richtung untereinander angeordnet. Sowohl die erste Elektrode wie auch die zweiten Elektroden sind auf der Aussenseite des Behälters angeordnet. Dritte bandförmige Elektroden sind in horizontaler Richtung innerhalb des Behälters angeordnet, wobei sie sich von der ersten zu den zweiten Elektroden erstrecken. In einer weiteren Ausführungsform sind lediglich die erste Elektrode und die zweiten Elektroden vorhanden, wobei der Behälter doppelwandig ausgeführt ist. Die erste Elektrode befindet sich auf der Aussenseite der äusseren Wand, die zweiten Elektroden sind auf der Aussenseite der inneren Wand angeordnet.

US 5 747 689 beschreibt einen Füllstandsensor, welcher teilweise in einen Flüssigkeitsbehälter eingetaucht ist. Er weist zwei parallel zueinander verlaufende und beabstandet voneinander angeordnete Platten auf. Eine der Platten ist segmentiert ausgebildet, wobei die einzelnen Segmente beabstandet zueinander angeordnet sind.

JP 2161322 zeigt einen kapazitiven Füllstandsensor mit zwei Elektroden, wobei eine erste Elektrode an der Aussenwand eines Behälters angebracht ist und die zweite Elektrode ein Rohrstück bildet, durch welches Wasser aus dem Behälter fliesst.

US 4 092 860 offenbart einen auf der Aussenseite eines Stutzens aufgebrachten kapazitiven Füllstandsensor, bestehend aus mehreren bandförmigen Elektroden. Dieser Sensor ist gegen aussen mit einer Teflonschicht geschützt.

DE 196 45 970 offenbart einen bandförmigen kapazitiven Füllstandsensor in Form eines Flachbandkabels. Die kapazitiven Sensorelemente sind durch Adern des Flachbandkabels gebildet. Der Sensor ist im Innern eines Behälters angeordnet.

WO 2009/098077 offenbart einen kapazitiven Füllstandsensor in einem Messbehälter, wobei der Sensor Segmente aufweist. Es können mehrere Sensorstreifen mit Segmenten verwendet werden.

Bei Drainagesystemen zum Absaugen von Körperflüssigkeiten befinden sich die Füllstandsensoren üblicherweise am oder im Fluidsammelbehälter. So offenbart WO 2008/119993 einen Fluidsammelbehälter mit einem auf der Aussenseite des Behälters angebrachten Füllstandsensor. Der Sensor umfasst mehrere bandförmige Platten, welche in vertikaler und horizontaler Richtung auf dem Behälter angeordnet sind.

EP 0 853 950 offenbart eine Drainagepumpeinheit mit einer Saugeinheit und einem Fluidsammelbehälter, bei welcher ein kapazitiver Füllstandsensor an der Saugeinheit angeordnet ist.

Fluidsammelbehälter können aus hygienischen Gründen nur eine begrenzte Zeit eingesetzt werden und sollten deshalb möglichst kostengünstig sein. Werden die Füllstandsensoren am Fluidsammelbehälter angebracht, erhöhen sich deren Kosten. Füllstandsensoren, welche an der Drainagepumpeinheit angebracht sind, weisen hingegen im Vergleich zu behälterseitigen Füllstandsensoren eine grössere Ungenauigkeit auf, insbesondere bei kleinen Füllmengen und insbesondere bei inhomogenen Flüssigkeiten.

### DARSTELLUNG DER ERFINDUNG

Es ist deshalb eine Aufgabe der Erfindung, eine Drainagepumpeinheit zu schaffen, welche die oben genannten Nachteile behebt.

Diese Aufgabe löst eine Drainagepumpeinheit mit den Merkmalen des Patentanspruchs 1 sowie ein Füllstandsensor mit den Merkmalen des Patentanspruchs 15.

Die erfindungsgemässe Drainagepumpeinheit zum Absaugen von Körperfluiden mittels einer Saugpumpe weist ein Saugpumpengehäuse und eine darin angeordnete Saugpumpe auf. Am Saugpumpengehäuse ist ein Fluidsammelbehälter lösbar befestigbar. Am Saugpumpengehäuse ist ferner ein kapazitiver Füllstandsensor angeordnet zur Detektion eines Füllstandes im Fluidsammelbehälter. Erfindungsgemäss weist der Füllstandsensor mindestens zwei Elektroden auf, welche beabstandet zueinander angeordnet sind und welche sich über eine gemeinsame Strecke erstrecken. Mindestens eine dieser Elektroden ist segmentiert ausgebildet. Mindestens eine der Elektroden einstückig und mindestens eine andere ist segmentiert ausgebildet. Es ist jedoch auch möglich, dass alle weiteren Elektroden segmentiert ausgebildet sind. Die einstückige Elektrode ist als Senderelektrode und die mindestens eine zweite segmentierte Elektrode ist als Empfängerelektrode ausgebildet. An die einstückige erste Elektrode ist von einem Modulator ein Anregungssignal anlegbar.

Dieses Anregungssignal ist kapazitiv in die segmentierte zweite Elektrode einkoppelbar.

Die Anordnung des Füllstandsensors am Saugpumpengehäuse ermöglicht es, die Kosten für die Fluidsammelbehälter möglichst tief zu halten.

Durch die Verwendung eines Füllstandssensors mit mindestens einer segmentierten Elektrode ist die Messgenauigkeit trotz Anordnung am Saugpumpengehäuse genügend gross. Die segmentierte Elektrode, insbesondere wenn sie als Empfängerelektrode ausgebildet ist, hilft Variationen im gemessenen Signalpegel zu kompensieren. Im Fall von mehreren Segmenten kann man das Verhältnis der Signale der verschiedenen Segmente zueinander messen. Dadurch lassen sich Beeinflussungen durch Flüssigkeitseigenschaften und durch Umgebungseffekte kompensieren, beispielsweise das Vorliegen von Metall in der Umgebung der Pumpe. Die segmentierte Elektrode erlaubt auch Messungen des Füllstandes in Zwischenhöhen, so dass nicht nur ein leerer bzw. ein voller Behälter detektiert werden kann.

Vorzugsweise erstrecken sich die Elektroden über annähernd eine gesamte befüllbare Höhe des Behälters. Dank dieses Füllstandssensors lassen sich auch Aussagen über die Füllgeschwindigkeit bzw. Füllrate des Fluidsammelbehälters machen.

In einer bevorzugten Ausführungsform sind mindestens zwei der Elektroden, vorzugsweise alle, in einer gemeinsamen Ebene nebeneinander angeordnet, wobei sie vorzugsweise in dieselbe Richtung ausgerichtet sind. Vorzugsweise sind sie in oder an einer Wand des Saugpumpengehäuses angeordnet, welche in bestimmungsgemässer Gebrauchslage dem Fluidsammelbehälter zugewandt ist. Vorzugsweise kontaktiert diese Wand den Fluidsammelbehälter oder es ist ein minimaler Luftspalt zwischen Wand und Fluidsammelbehälter vorhanden.

In einer anderen Ausführungsform ist eine erste Elektrode auf einer einer zweiten Elektrode gegenüberliegenden Seite angeordnet, wobei sie einander zugewandt sind. Vorzugsweise sind dabei beide Elektroden in je einer Wand angeordnet, wobei beide Wände dem Fluidsammelbehälter zugewandt sind. Insbesondere kontaktieren diese Wände den Fluidsammelbehälter, oder es liegt nur ein minimaler Luftspalt dazwischen.

In einer bevorzugten Ausführungsform ist die segmentierte Elektrode in Segmente unterteilt, welche voneinander elektrisch isoliert, mit einem Abstand von weniger als 1 mm aneinander anschliessend angeordnet sind. Dieses praktisch abstandslose Aneinanderreihen der Segmente erhöht die Genauigkeit der Füllstandsdetektion. Vorzugsweise ist diese Elektrode in mehr als drei oder vier, insbesondere in genau acht Segmente unterteilt. Die Segmente sind vorzugsweise alle gleich hoch. Sie können jedoch auch unterschiedliche Höhen aufweisen.

In einer bevorzugten Ausführungsform ist eine Elektronik vorhanden, mittels welcher eine erste Elektrode oszillierend anregbar ist, insbesondere in einer Sinusfunktion. Die Anregung dieser ersten Elektrode erfolgt vorzugsweise mit einer Frequenz im Bereich von 4 bis 5 MHz. Es hat sich gezeigt, dass in diesem Frequenzbereich ein Messsignal erhalten wird, welches praktisch unabhängig von der Art des im Fluidsammelbehälter gesammelten Sekrets ist. Insbesondere eine Frequenz von mindestens annähernd 4 MHz oder von 4.75 MHz hat sehr gute Resultate erzielt.

Um eine Beeinflussung des Messsignals durch Flüssigkeitseigenschaften und Umgebungseffekte auch bei niedrigen Füllständen möglichst optimal zu kompensieren, lässt sich zudem eine weitere Anregung verwenden. Diese beträgt vorzugsweise annähernd 100 kHz.

In einer bevorzugten Ausführungsform sind die Elektroden bandförmig ausgebildet. Die Segmente der segmentierten Elektrode sind dabei vorzugsweise in einer Linie übereinander angeordnet. Sie können jedoch auch versetzt zueinander übereinander angeordnet sein.

Vorzugsweise sind die Elektroden kupferbeschichtete Leiterplatten.

Der Füllstandssensor ist einfach in das Saugpumpengehäuse einbaubar, wenn die Elektroden auf derselben Leiterplatine angeordnet sind. Vorzugsweise befindet sich auch die Elektronik des Sensors, inkl. einem Mikroprozessor zur Auswertung des Signals, auf dieser Leiterplatine.

In einer einfachen Ausführungsform sind die zwei Elektroden auf einer Aussenseite des Saugpumpengehäuses angeordnet. Es kann auch eine der Elektroden auf der Aussenseite und die andere auf einer Innenseite angeordnet sein. Vorzugsweise sind die Elektroden jedoch einzeln oder auf der gemeinsamen Leiterplatine an einer Innenwand des Saugpumpengehäuses, d.h. auf der Innenseite, angeordnet. Dies schützt die sensitiven Elektrodenflächen vor äusserer Beanspruchung, beispielsweise beim Wechseln des Fluidsammelbehälters.

Die Montage ist vereinfacht und der Sensor optimal geschützt, wenn die Innenwand des Saugpumpengehäuses eine Vertiefung zur Aufnahme der Elektroden aufweist, wobei die Vertiefung so bemessen ist, dass die Elektroden der Innenwand in Richtung Behälterinnenseite nicht vorstehen. Vorzugsweise fluchtet die Rückseite der Elektroden oder der Leiterplatine mit der Randfläche der Innenwand.

Vorteilhaft an der erfindungsgemässen Drainagepumpeinheit ist ferner, dass sich ein und derselbe Füllstandsensor ohne spezielle Anpassungen für unterschiedliche geformte Behälter bzw. für Behälter mit unterschiedlichen Grössen verwenden lässt.

Weitere Ausführungsformen sind in den abhängigen Ansprüchen angegeben.

### KURZE BESCHREIBUNG DER ZEICHNUNGEN

Bevorzugte Ausführungsformen der Erfindung sind im Folgenden anhand der Zeichnungen beschrieben, die lediglich zur Erläuterung dienen und nicht einschränkend auszulegen sind. In den Zeichnungen zeigen:
- Figur 1: eine perspektivische schematische Darstellung einer erfindungsgemässen Drainagepumpeinheit mit offenem Gehäuse in einer ersten Ausführungsform;
- Figur 2: eine weitere perspektivische Darstellung der Drainagepumpeinheit gemäss Figur 1 mit teilweise weg geschwenktem Fluidsammelbehälter;
- Figur 3: eine perspektivische schematische Darstellung einer erfindungsgemässen Drainagepumpeinheit mit offenem Gehäuse in einer zweiten Ausführungsform;
- Figur 4: einen Querschnitt durch die Drainagepumpeinheit gemäss Figur 3 ohne Fluidsammelbehälter;
- Figur 5: ein Schaltbild der erfindungsgemässen Vorrichtung;
- Figur 6: eine Darstellung eines Grundprinzips der erfindungsgemässen Drainagepumpeinheit gemäss der ersten und zweiten Ausführungsform und
- Figur 7: eine Darstellung eines Grundprinzips der erfindungsgemässen Drainagepumpeinheit gemäss einer dritten Ausführungsform.

### BESCHREIBUNG BEVORZUGTER AUSFÜHRUNGSFORMEN

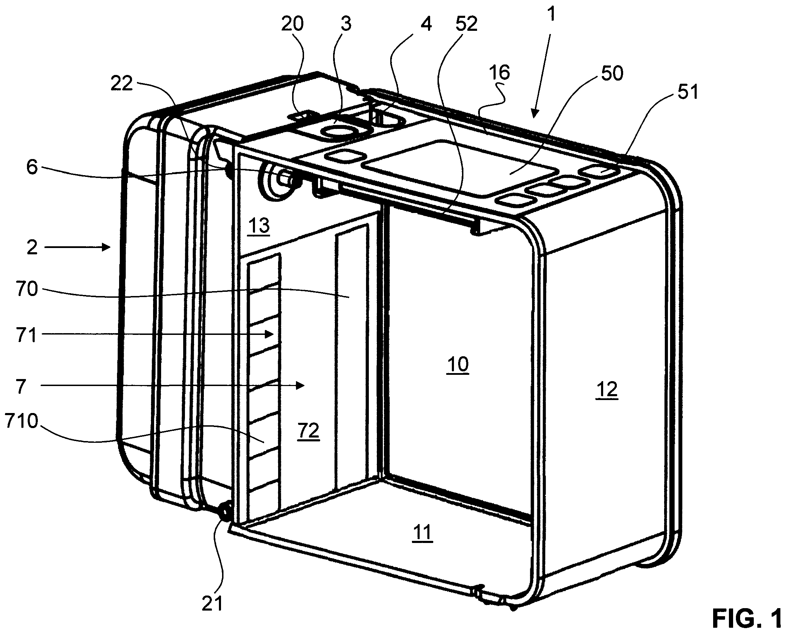

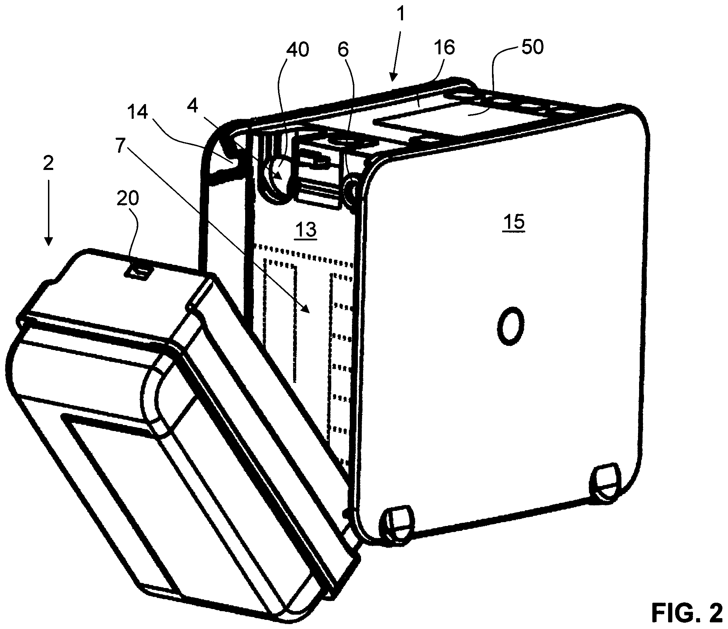

In den Figuren 1 und 2 ist eine Drainagepumpeinheit dargestellt, wie sie im Grundaufbau beispielsweise aus WO 2007/128156 und WO 2008/141471 bekannt ist. Diese Pumpeinheiten dienen zum Absaugen von Körperflüssigkeiten bzw. -fluiden im medizinischen Bereich und sind vorzugsweise tragbar ausgebildet. Das heisst, der Patient kann sich während des Abpumpens frei bewegen.

Die Drainagepumpeinheit weist ein Saugpumpengehäuse 1 mit einer Rückwand 10, einem Boden 11, zwei Seitenwänden 12, 13, einer vorderen Wand 15 und einer oberen Wand 16 auf. Das Saugpumpengehäuse 1 ist in diesem Beispiel quaderförmig ausgebildet, wobei mindestens eine seitliche äussere Gehäusewand, hier die zweite Seitenwand 13, im Wesentlichen plan ausgebildet ist. Das Saugpumpengehäuse 1 kann jedoch auch eine andere Form aufweisen.

Das Gehäuse 1 kann tragbar ausgebildet sein, beispielsweise mittels eines nicht dargestellten Traggriffs. Vorzugsweise weist das Gehäuse 1 alternativ oder zusätzlich zum Traggriff eine Standfläche oder Standfüsse auf, so dass das Gehäuse in der abgebildeten Lage auf einem Tisch oder einer anderen geeigneten Standfläche aufgestellt werden kann. Nachfolgend angegebene Richtungsangaben sind in dieser stehenden Position des Gehäuses zu verstehen.

Im Gehäuse 1 ist eine nicht dargestellte Saug- oder Vakuumpumpe angeordnet. Diese Saugpumpe ist über am Gehäuse 1 angeordnete Bedientasten 51 bedienbar. Anstelle von Bedientasten können auch Schalter oder Bedienfelder eingesetzt sein. Ein Display 50 zeigt notwendige Informationen zum Betrieb der Pumpe bzw. der Drainagepumpeinheit an. Eine Elektronik 52 zur Steuerung der Pumpe und zur Steuerung bzw. Auswertung allfälliger weiterer in der Drainagepumpeinheit vorhandener Elemente ist ebenfalls im Gehäuse 1 angeordnet und mit den Tasten 51 und dem Display 50 verbunden.

An der zweiten Seitenwand 13 stehen die vordere Wand 15 und die Rückwand 10 seitlich vor und bilden zusammen mit der zweiten Seitenwand 13 eine Aufnahme für einen Fluidsammelbehälter 2.

Dieser Fluidsammelbehälter 2 ist vorzugsweise im Wesentlichen quaderförmig ausgebildet. Das Saugpumpengehäuse 1 und der Fluidsammelbehälter 2 sind vorzugsweise aus Kunststoff gefertigt.

Der Fluidsammelbehälter 2 ist lösbar am Saugpumpengehäuse 1 befestigt. Vorzugsweise wird er dabei eingeschwenkt und rastet in dieser Position ein. Hierzu weisen der vorstehende Teil der vorderen Wand 15 und der Rückwand 10 obere und untere Kulissenführungen 14 auf, in welche obere und untere Einrastbolzen 21, 22 des Fluidsammelbehälters 2 eingreifen. Der Fluidsammelbehälter 2 weist zwei einander gegenüberliegende obere und zwei einander gegenüberliegende untere Einrastbolzen 21, 22 auf, welche alle derselben äusseren Seitenwand des Behälters 2 vorstehen. Mit dieser Seitenwand liegt der Fluidsammelbehälter 2 an der zweiten Seitenwand 13 des Saugpumpengehäuses 1 an, wobei ein kleiner Luftspalt vorhanden sein kann. Die Seitenwand des Fluidsammelbehälters 2 ist vorzugsweise ebenfalls im Wesentlichen plan ausgebildet.

Der Sammelbehälter 2 weist eine Ausnehmung 20 auf, in welche ein am Gehäuse 1 angeordnetes Verriegelungselement 3, hier ein Einrasthaken, einrastet und so den Sammelbehälter 2 in seiner Lage am Gehäuse 1 lösbar fixiert. Anstelle von Kulissenführungen, Einrastbolzen und Einrasthaken kann die Befestigung des Sammelbehälters 2 und seine Fixierung auch mit anderen Mitteln erfolgen. Auch die körperliche Gestaltung des Gehäuses 1 und des Sammelbehälters 2 im Bereich ihrer Angrenzung aneinander kann anders ausgebildet sein.

In der zweiten Seitenwand 13 des Gehäuses 1 ist ein gehäuseseitiger Vakuumanschluss 6 vorhanden. Vorzugsweise ist er in einem oberen Bereich der zweiten Seitenwand 13 angeordnet. Der Vakuumanschluss 6 weist in diesem Beispiel die Form eines Stutzens auf, welcher in eine hier nicht dargestellte entsprechende Öffnung des Fluidsammelbehälters 2 eingreift. Das freie Ende des Stutzens 6 fluchtet dabei mit der zweiten Seitenwand 13 des Gehäuses 1 oder ist sogar relativ zu ihr in Richtung Gehäuse 1 zurückversetzt. Vorzugsweise steht der Stutzen 6 somit nicht vor. Damit die durch den Stutzen 6 gebildete Vakuumverbindung zwischen Gehäuse 1 und Behälter 2 dicht ist, weist der Stutzen 6 des Gehäuses 1 und oder die Öffnung des Behälters 2 einen hier nicht dargestellten Dichtring auf. Damit im Behälter 2 gesammeltes abgesaugtes Sekret nicht in das Gehäuse 1 gelangen kann, ist der Vakuumanschluss 6 und/oder die zugehörige Öffnung des Behälters 2 vorzugsweise mit einem oder mehreren Filtern versehen.

Im oberen Bereich der zweiten Seitenwand 13, an der dem Vakuumanschluss 6 gegenüberliegenden Seite, ist ein gehäuseseitiger Sekretanschluss bzw. eine Ausnehmung 4 zur Anbringung eines derartigen Anschlusses vorhanden. Hier lässt sich ein pumpenseitiges Anschlussteil eines patientenseitigen Drainageschlauchs einstecken, wie dies in WO 2008/141471 offenbart ist. Dieser Sekretanschluss wird wie der Vakuumanschluss 6 dichtend mit einer entsprechenden Öffnung des Fluidsammelbehälters 2 verbunden, wenn der Fluidsammelbehälter 2 am Gehäuse 1 befestigt, hier eingeschwenkt, wird. Es ist jedoch auch möglich, die Verbindung zwischen Behälter 2 und patientenseitigem Drainageschlauch auf andere Art und Weise zu erstellen, beispielsweise kann der Schlauch auch direkt bzw. über geeignete Kopplungsteile im Behälter 2 einsteckbar sein. Ferner ist ein gehäuseseitiger Serviceeingang 40 vorhanden, welcher mit einem zum Patienten führenden Serviceschlauch verbindbar ist.

Über den Vakuumanschluss 6 wird mittels der Saugpumpe ein Unterdruck im Behälter 2 erzeugt. Dank diesem Unterdruck wird ein Fluid oder Sekret aus der Kavität des Patienten durch den Drainageschlauch in den Behälter 2 gesaugt und dort gesammelt.

Die erfindungsgemässe Vorrichtung umfasst mindestens einen kapazitiven Füllstandsensor 7, mittels welchem der Füllstand im Fluidsammelbehälter 2 feststellbar ist. Der Füllstandsensor 7 ist in der zweiten Seitenwand 13, welche dem Fluidsammelbehälter 2 zugewandt ist, angeordnet.

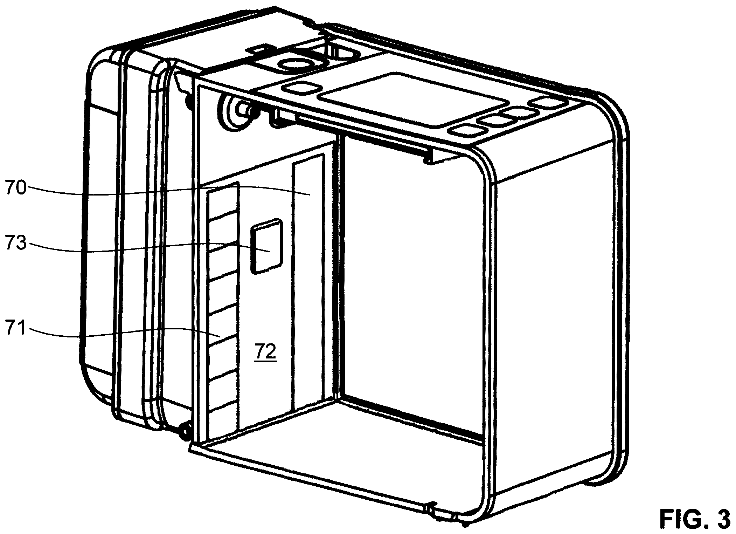

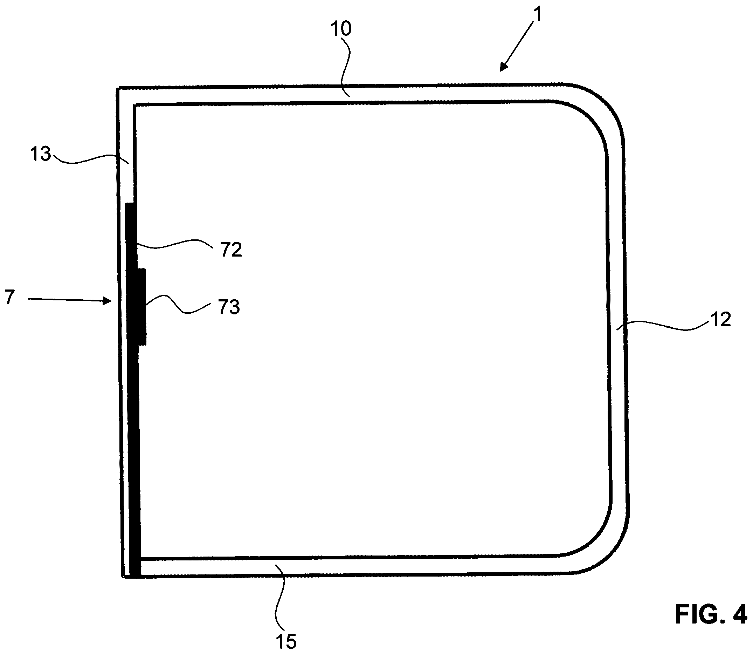

Er weist mindestens zwei, hier genau zwei Elektroden auf. Eine erste Elektrode 70 bildet einen Sender, eine zweite Elektrode 71 bildet einen Empfänger zum Empfang des vom Sender ausgesendeten Signals. Es wird ein Ladungstransfer von Sender- zur Empfängerelektrode ermittelt. Die zwei Elektroden 70, 71 verlaufen parallel beabstandet zueinander. In diesem Beispiel sind sie in einer gemeinsamen Ebene angeordnet. Ihre Sender- bzw. Empfängerflächen sind dabei in dieselbe Richtung gerichtet, nämlich zum Fluidsammelbehälter 2 hin. Dabei sind die Elektroden 70, 71 vorzugsweise auf der Innenseite des Gehäuses 1 angeordnet, so dass die Seitenwand 13 zwischen sensitiven Elektrodenflächen und Fluidsammelbehälter 2 liegt. Die Seitenwand 13 kann in diesem Bereich verdünnt ausgebildet sein oder ein Fenster mit einem geeigneten dielektrischen Material aufweisen. Vorzugsweise ist eine Vertiefung auf der Innenseite der zweiten Seitenwand 13 vorhanden, in welche die Elektroden 70, 71 gemeinsam oder einzeln eingelegt sind. In diesem Beispiel sind die Elektroden 70, 71 auf einer gemeinsamen Leiterplatine 72 angeordnet, welche in die Vertiefung so eingelassen ist, dass die Rückseiten der Leiterplatine 72 und/oder der Elektroden 70, 71 mit der Innenseite der zweiten Seitenwand 13 fluchten. Im Ausführungsbeispiel gemäss den Figuren 3 und 4 ist auch ein Mikroprozessor 73 zur Betreibung des Füllstandssensors 7 auf dieser Leiterplatine 72 angeordnet. Dieser Mikroprozessor 73 ist vorzugsweise mit der restlichen Steuerungselektronik 52 der Saugpumpeneinheit verbunden.

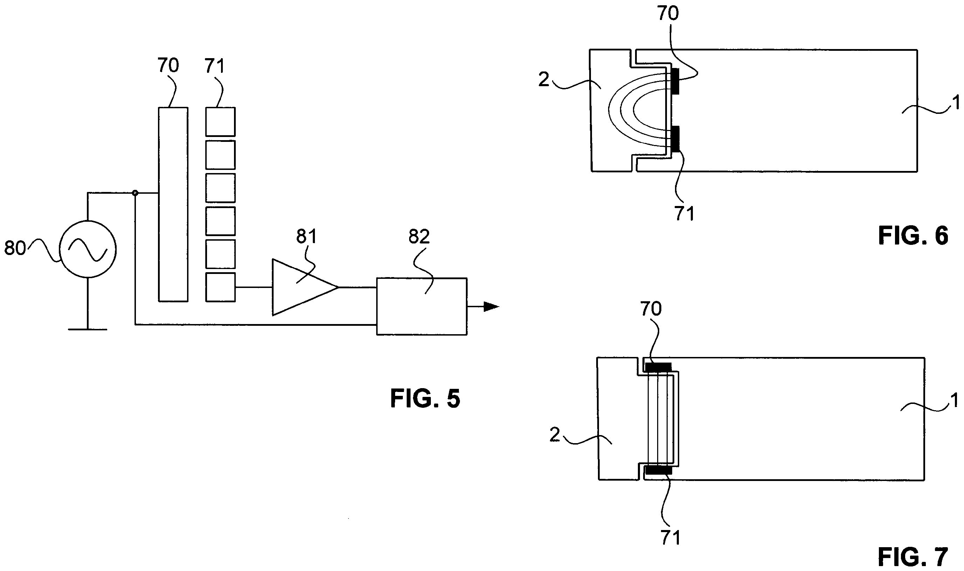

In Figur 6 ist die innenseitige Anordnung der Elektroden 70, 71 nochmals schematisch dargestellt. In Figur 7 ist eine von weiteren möglichen Anordnungen gezeigt. Hier sind die zwei Elektroden 70, 71 nach wie vor auf der Innen- oder Aussenseite des Gehäuses 1 angeordnet, jedoch derart, dass sie einander gegenüberliegen. Im vorliegenden Gehäuse 1 kann dies erreicht werden, indem die Elektroden 70, 71 nicht in der zweiten Seitenwand 13, sondern an oder in den der Seitenwand 13 vorstehenden Teilen der vorderen Wand 15 und der Rückwand 10 angeordnet werden.

Die zwei Elektroden 70, 71 sind jeweils bandförmig ausgebildet mit einer Länge, einer Breite und einer Dicke. Die Dicke weist vorzugsweise die kleinste Dimension auf, wobei sie ein Vielfaches kleiner als die Breite und Länge ist. Die Breite der Elektroden 70, 71 ist ebenfalls wesentlich kleiner als die Länge, vorzugsweise um mindestens eine Dimension. Vorzugsweise sind die zwei Elektroden 70, 71 in ihrer Gesamtlänge, Breite und Dicke identisch ausgebildet. Typische Werte liegen bei: Dicke 35 µm; Länge 10 cm; Breite 2 cm.

Vorzugsweise sind die zwei Elektroden 70, 71 aus kupferbeschichteten Leiterplatten gebildet. Sind sie auf einer gemeinsamen Platine angeordnet, so kann diese mit einer Kupferschicht versehen sein.

Die zwei Elektroden 70, 71 erstrecken sich über einen gewünschten Messbereich des Fluidsammelbehälters 2. Vorzugsweise erstrecken sie sich annähernd über die gesamte mögliche Füllhöhe des Fluidsammelbehälters 2. Dies entspricht in diesem Beispiel einem wesentlichen Teil der Höhe der zweiten Seitenwand 13. Typische Längen betragen 9 cm bis 15 cm. Vorzugsweise sind die zwei Elektroden 70, 71 mit ihrer Länge in vertikaler Richtung verlaufend angeordnet.

Der Abstand der zwei Elektroden 70, 71 voneinander ist so gross wie möglich, das heisst, sie befinden sich an den äusseren Rändern der zweiten Seitenwand 13 bzw. an den äusseren Rändern des Fluidsammelbehälters 2. Typische Abstände betragen 25 bis 40 mm.

Die erste Elektrode 70 ist einstückig ausgebildet. Die zweite Elektrode 71 ist segmentiert. Vorzugsweise ist sie in identisch grosse Segmente 710 mit gleicher Höhe und vorzugsweise auch gleicher Breite unterteilt. Die Anzahl der Segmente 710 ist beliebig. Vorzugsweise sind es mindestens vier Segmente 710. Gute Resultate wurden mit acht Segmenten 710 erzielt. Die Segmente 710 sind vorzugsweise vertikal übereinander angeordnet. Sie können in dieser Anordnung in einer Linie oder zueinander versetzt angeordnet sein. Die Segmente 710 reihen sich möglichst ohne Abstand aneinander, wobei der Abstand vorzugsweise lediglich durch eine elektrische Isolation gegeben ist. Dieser Abstand ist somit relativ klein und beträgt vorzugsweise maximal 1 mm.

In Figur 5 ist das Messprinzip des erfindungsgemässen Füllstandsensors 7 schematisch dargestellt. An die erste Elektrode 70 wird von einem Modulator 80 ein Anregungssignal angelegt, welches kapazitiv in die zweite Elektrode 71 eingekoppelt wird. Dieses eingekoppelte Signal wird über einen Vorverstärker 81 als Messsignal einem Demodulator 82 zugeführt. Die Amplitude des Messsignals wird durch das Teilerverhältnis des kapazitiven Spannungsteilers bestimmt, welcher aus der Kopplungskapazität einerseits und der Eingangskapazität des Sensorverstärkers und weiterer parasitärer Kapazitäten andererseits besteht. Die Kopplungskapazität von der Sender- zur Empfängerelektrode 70, 71 hängt von deren Abstand und den dazwischen befindlichen Medien ab. Da die relative Permeabilität einer wässrigen Lösung sehr viel höher ist als diejenigen von Luft und Kunststoff, lässt sich der Füllstand im Behälter relativ genau detektieren.

Vorzugsweise wird als Anregungssignal ein Sinussignal angelegt. Die angelegten ersten Frequenzen liegen vorzugsweise im Bereich von 4 bis 5 MHz, insbesondere von annähernd 4 mHz oder von 4.75 MHz. In einer bevorzugten Variante wird zusätzlich, insbesondere bei niedrigen Füllständen, eine zweite Messfrequenz von annähernd 100 kHz an die erste Elektrode 70 angelegt. Auch dieses Signal ist vorzugsweise ein Sinussignal. Die zwei unterschiedlichen Anregungssignale werden vorzugsweise alternierend angelegt, können aber auch überlagert werden.

Die Signalamplitude dieser tiefen Frequenz ist stark von den Eigenschaften, insbesondere von der Zusammensetzung, des Fluids im Fluidsammelbehälter abhängig. Durch Vergleich der Signalamplituden bei einer Anregung mit der ersten hohen und der zweiten tiefen Frequenz ergeben sich Rückschlüsse auf das Fluid im Sammelbehälter. Damit können einerseits Medieneinflüsse auch bei geringen Füllständen kompensiert werden. Andererseits kann der erfindungsgemässe Fluidsensor 7 zur Detektion von Eigenschaften, insbesondere der Art bzw. Zusammensetzung des Fluids, verwendet werden. Diese Angaben können beispielsweise für die Art und Weise, wie bzw. ob die Pumpe betrieben werden soll und/oder für die Behandlung des Patienten verwendet werden.

Alternativ kann anstelle eines Sinussignals auch ein Rechteck- oder ein Dreiecksignal zur Anregung verwendet werden. Auch eine Phasenmessung ist möglich.

Die oben beschriebene Anregung mit zwei unterschiedlichen Signalen lässt sich auch als erfindungsgemässes Verfahren mit anderen Füllstandsensoren einsetzen.

Zusätzlich kann in der erfindungsgemässen Drainagepumpeinheit, insbesondere im Gehäuse1, ein Neigungssensor angeordnet sein. Mittels dieser Informationen lässt sich das Messsignal korrigieren, so dass auch bei geneigter Lage des Fluidsammelbehälters eine der Wirklichkeit entsprechender Aussage über seinen Füllstand erhalten wird.

Die erfindungsgemässe Drainagepumpeinheit ermöglicht dank der segmentierten Elektrode die Herstellung von kostengünstigen Fluidsammelbehältern und gewährleistet trotzdem eine grosse Messgenauigkeit.

**BEZUGSZEICHENLISTE**

| | | | |
|---|---|---|---|
| 1 | Saugpumpengehäuse | 40 | Serviceeingang |
| 10 | Rückwand | | |
| 11 | Boden | 50 | Display |
| 12 | erste Seitenwand | 51 | Bedientasten |
| 13 | zweite Seitenwand | 52 | Steuerungselektronik |
| 14 | Kulissenführung | | |
| 15 | Vordere Wand | 6 | Vakuumanschluss |
| 16 | obere Wand | | |
| | | 7 | Füllstandsensor |
| 2 | Fluidsammelbehälter | 70 | erste Elektrode |
| 20 | Ausnehmung | 71 | zweite Elektrode |
| 21 | unterer Einrastbolzen | 710 | Elektrodensegment |
| 22 | oberer Einrastbolzen | 72 | Leiterplatte |
| | | 73 | Mikroprozessor |
| 3 | Verriegelungselement | | |
| | | 80 | Modulator |
| 4 | Ausnehmung für Drainageanschluss | 81 | Vorverstärker |
| | | 82 | Demodulator |

## Patentansprüche

1. Drainagepumpeinheit zum Absaugen von Körperfluiden mittels einer Saugpumpe, wobei die Drainagepumpeinheit ein Saugpumpengehäuse (1) und eine darin angeordnete Saugpumpe aufweist, wobei am Saugpumpengehäuse (1) ein Fluidsammelbehälter (2) lösbar befestigbar ist und wobei am Saugpumpengehäuse (1) ein kapazitiver Füllstandsensor (7) angeordnet ist zur Detektion eines Füllstandes im Fluidsammelbehälter (2), **dadurch gekennzeichnet, dass** der Füllstandsensor (7) mindestens zwei Elektroden (70, 71) aufweist, welche beabstandet zueinander angeordnet sind und sich über eine gemeinsame Strecke erstrecken, dass eine erste der mindestens zwei Elektroden (70) einstückig und mindestens eine zweite dieser Elektroden (71) segmentiert ausgebildet ist und dass die einstückige erste Elektrode (70) als Senderelektrode und die segmentierte zweite Elektrode (71) als Empfängerelektrode ausgebildet ist, wobei an die einstückige erste Elektrode (70) ein Anregungssignal von einem Modulator (80) anlegbar ist.

2. Drainagepumpeinheit nach Anspruch 1, wobei das Anregungssignal kapazitiv in die segmentierte zweite Elektrode (71) einkoppelbar ist.

3. Drainagepumpeinheit nach einem der Ansprüche 1 oder 2, wobei sich die Elektroden (70, 71) über annähernd eine gesamte befüllbare Höhe des Fluidsammelbehälters (2) erstrecken.

4. Drainagepumpeinheit nach einem der Ansprüche 1 bis 3, wobei die mindestens zwei Elektroden (70, 71) in einer gemeinsamen Ebene nebeneinander angeordnet sind oder wobei sie einander gegenüberliegend angeordnet sind.

5. Drainagepumpeinheit nach einem der Ansprüche 1 bis 4, wobei die mindestens zwei Elektroden (70, 71) in oder an einer Wand (13) des Saugpumpengehäuses (1) angeordnet sind, welche in bestimmungsgemässer Gebrauchslage dem Fluidsammelbehälter (2) zugewandt ist.

6. Drainagepumpeinheit nach einem der Ansprüche 1 bis 5, wobei die mindestens eine segmentierte Elektrode (71) in Segmente (710) unterteilt ist, welche voneinander elektrisch isoliert, mit einem Abstand von weniger als 1 mm aneinander anschliessend angeordnet sind.

7. Drainagepumpeinheit nach einem der Ansprüche 1 bis 6, wobei eine Elektronik (73) vorhanden ist, mittels welcher eine der Elektroden (70) mit einem oszillierenden ersten Signal anregbar ist, insbesondere in einer Sinusfunktion, wobei die Anregung vorzugsweise mit einer Frequenz im Bereich von 4 bis 5 MHz, insbesondere von mindestens annähernd 4 MHz und von 4.75 MHz, erfolgt.

8. Drainagepumpeinheit nach Anspruch 7, wobei die Elektronik (73) zur Anregung dieser anregbaren Elektrode (70) mit einem oszillierenden zweiten Signal ausgebildet ist, wobei diese Anregung vorzugsweise mit einer Frequenz im Bereich von annähernd 100 kHz erfolgt.

9. Drainagepumpeinheit nach einem der Ansprüche 1 bis 8, wobei die mindestens eine segmentierte Elektrode (71) in mehr als drei, insbesondere in genau acht Segmente (710) unterteilt ist.

10. Drainagepumpeinheit nach einem der Ansprüche 1 bis 9, wobei die mindestens zwei Elektroden (70, 71) bandförmig ausgebildet sind.

11. Drainagepumpeinheit nach einem der Ansprüche 1 bis 10, wobei die mindestens zwei Elektroden (70, 71) kupferbeschichtete Leiterplatten sind.

12. Drainagepumpeinheit nach einem der Ansprüche 1 bis 11, wobei die mindestens zwei Elektroden (70, 71) auf derselben Leiterplatine (72) angeordnet sind.

13. Drainagepumpeinheit nach einem der Ansprüche 1 bis 12, wobei die mindestens zwei Elektroden (70, 71) an einer Innenwand (13) des Saugpumpengehäuses (1) angeordnet sind.

14. Drainagepumpeinheit nach Anspruch 13, wobei die Innenwand (13) des Saugpumpengehäuses (1) eine Vertiefung zur Aufnahme der mindestens zwei Elektroden (70, 71) aufweist, wobei die Vertiefung so bemessen ist, dass die mindestens zwei Elektroden (70, 71) der Innenwand (13) in Richtung Behälterinnenseite nicht vorstehen.

15. Füllstandsensor (7) zur Verwendung in einer Drainagepumpeinheit gemäss einem der Ansprüche 1 bis 14, **dadurch gekennzeichnet, dass** der Füllstandsensor (7) mindestens zwei Elektroden (70, 71) aufweist, welche in einer gemeinsamen Ebene nebeneinander, jedoch beabstandet zueinander angeordnet sind und welche sich über eine gemeinsame Strecke erstrecken, dass eine erste der mindestens zwei Elektroden (70) einstückig und mindestens eine zweite dieser Elektroden (71) segmentiert ausgebildet ist und dass die einstückige erste Elektrode (70) als Senderelektrode und die segmentierte zweite Elektrode (71) als Empfängerelektrode ausgebildet ist, wobei an die einstückige erste Elektrode (70) ein Anregungssignal von einem Modulator (80) anlegbar ist.

16. Füllstandsensor nach Anspruch 15, wobei das Anregungssignal kapazitiv in die segmentierte zweite Elektrode (71) einkoppelbar ist.

## Claims

1. Drainage pump unit for aspirating body fluids by means of a suction pump, wherein the drainage pump unit has a suction pump housing (1) and a suction pump arranged in the latter, wherein a fluid collection container (2) can be secured releasably on the suction pump housing (1), and wherein a capacitive filling level sensor (7) is arranged on the suction pump housing (1) for the purpose of detecting a filling level in the fluid collection container (2), **characterized in that** the filling level sensor (7) has at least two electrodes (70, 71), which are arranged at a distance from each other and extend along a common path, that a first of the at least two electrodes (70) is in one piece, and at least a second of these electrodes (71) is segmented and that the one-piece first electrode (70) is designed as transmitter electrode and the segmented second electrode (71) is designed as receiver electrode, wherein an excitation signal can be applied by a modulator (80) to the one-piece first electrode (70).

2. Drainage pump unit according to claim 1, wherein the excitation signal can be coupled capacitively into the segmented second electrode (71).

3. Drainage pump unit according to one of Claims 1 or 2, wherein the electrodes (70, 71) extend along approximately an entire fillable height of the fluid collection container (2).

4. Drainage pump unit according to one of Claims 1 or 3, wherein the at least two electrodes (70, 71) are arranged next to each other in a common plane, or wherein they are arranged lying opposite each other.

5. Drainage pump unit according to one of Claims 1 to 4, wherein the at least two electrodes (70, 71) are arranged in or on a wall (13) of the suction pump housing (1), which wall (13) faces towards the fluid collection container (2) in the intended position of use.

6. Drainage pump unit according to one of Claims 1 to 5, wherein the at least one segmented electrode (71) is divided into segments (710), which are electrically insulated from one another and are arranged with a spacing of less than 1 mm between one another.

7. Drainage pump unit according to one of Claims 1 to 6, wherein an electronics system (73) is present, by means of which one of the electrodes (70) can be excited with an oscillating first signal, particularly in a sinusoidal function, wherein the excitation preferably takes place at a frequency in the range of 4 to 5 MHz, particularly of at least approximately 4 MHz and 4.75 MHz.

8. Drainage pump unit according to Claim 7, wherein the electronics system (73) is designed to excite this excitable electrode (70) with an oscillating second signal, wherein this excitation preferably takes place at a frequency in the range of approximately 100 kHz.

9. Drainage pump unit according to one of Claims 1 to 8, wherein the at least one segmented electrode (71) is divided into more than three segments (710), particularly into precisely eight segments (710).

10. Drainage pump unit according to one of Claims 1 to 9, wherein the at least two electrodes (70, 71) are band-shaped.

11. Drainage pump unit according to one of Claims 1 to 10, wherein the at least two electrodes (70, 71) are copper-coated circuit boards.

12. Drainage pump unit according to one of Claims 1 to 11, wherein the at least two electrodes (70, 71) are arranged on the same printed circuit board (72).

13. Drainage pump unit according to one of Claims 1 to 12, wherein the at least two electrodes (70, 71) are arranged on an inner wall (13) of the suction pump housing (1).

14. Drainage pump unit according to Claim 13, wherein the inner wall (13) of the suction pump housing (1) has a depression for receiving the at least two electrodes (70, 71), said depression being so dimensioned that the at least two electrodes (70, 71) do not protrude from the inner wall (13) in the direction of the container interior.

15. Filling level sensor (7) for use in a drainage pump unit according to one of Claims 1 to 14, **characterized in that** the filling level sensor (7) has at least two electrodes (70, 71), which are arranged next to each other in a common plane, but at a distance from each other, and extend along a common path, that a first of the at least two electrodes (70) is in one piece and at least a second of these electrodes (71) is segmented and that the one-piece first electrode (70) is a transmitter electrode and the segmented second electrode (71) is a receiver electrode, wherein an excitation signal can be applied by a modulator (80) to the one-piece first electrode (70).

16. Filling level sensor according to claim 15, wherein the excitation signal can be coupled capacitively into the segmented second electrode (71).

## Revendications

1. Une unité de pompe de drainage pour l'aspiration de fluides corporels au moyen d'une pompe d'aspiration, l'unité de pompe de drainage comprenant un boîtier de pompe d'aspiration (1) et une pompe d'aspiration disposée dans celui-ci, où un récipient de collecte de fluide (2) peut être fixé sur le boîtier de pompe d'aspiration (1) de manière amovible et où un capteur de niveau capacitif (7) est disposé sur le boîtier de pompe d'aspiration (1) pour la détection d'un niveau dans le récipient de collecte de fluide (2), **caractérisée en ce que** le capteur de niveau (7) comporte au moins deux électrodes (70, 71), lesquelles sont disposées de manière espacée l'une par rapport à l'autre et s'étendent sur une distance commune, qu'une première électrode des au moins deux électrodes (70) est fabriquée d'une seule pièce et au moins une deuxième électrode de ces électrodes (71) est formée de manière segmentée et que la première électrode (70) fabriquée d'une seule pièce est formée comme électrode d'émission et la deuxième électrode formée de manière segmentée (71) est formée comme électrode de réception, où un signal d'excitation d'un modulateur (80) peut être appliqué à la première électrode (70) fabriquée d'une seule pièce.

2. Une unité de pompe de drainage selon la revendication 1, où le signal d'excitation capacitif peut être couplé dans la deuxième électrode formée de manière segmentée (71).

3. Une unité de pompe de drainage selon l'une des revendications 1 ou 2, où les électrodes (70, 71) s'étendent approximativement sur le niveau entier réalisable du récipient de collecte de fluide (2).

4. L'unité de pompe de drainage selon une des revendications 1 à 3, où les au moins deux électrodes (70, 71) sont disposées dans un plan commun l'une à cote de l'autre, ou où elles sont disposées l'une opposé à l'autre.

5. L'unité de pompe de drainage selon une des revendications 1 à 4, où les au moins deux électrodes (70, 71) sont disposées dans ou sur une paroi (13) du boîtier de pompe d'aspiration (1), laquelle fait face au récipient de collecte de fluide (2) dans son orientation conforme.

6. L'unité de pompe de drainage selon une des revendications 1 à 5, où l'au moins une électrode (71) formée de manière segmentée est subdivisée en segments (710), lesquels sont isolés de manière électrique l'un par rapport à l'autre et disposés en se raccordant l'un à l'autre avec un espace de moins de 1 millimètre.

7. L'unité de pompe de drainage selon une des revendications 1 à 6, où une électronique (73) est prévue, au moyen une des électrodes (70) peut être excitée à l'aide d'un premier signal oscillant, en particulier d'une fonction sinusoïdale, où l'excitation est effectuée de manière préférable avec une fréquence dans le domaine de 4 à 5 MHz, en particulier d'au moins approximativement 4 MHz et de 4,75 MHz.

8. L'unité de pompe de drainage selon la revendication 7, où l'électronique (73) est formée avec un deuxième signal oscillant pour l'excitation de cette électrode (70) excitable, où cette excitation est effectuée de manière préférable avec une fréquence dans le domaine d'approximativement 100 kHz.

9. L'unité de pompe de drainage selon une des revendications 1 à 8, où l'au moins une électrode (71) formée de manière segmentée est subdivisée en plus que trois, en particulier exactement huit segments (710).

10. L'unité de pompe de drainage selon une des revendications 1 à 9, où les au moins deux électrodes (70, 71) sont formées en forme de bande.

11. L'unité de pompe de drainage selon une des revendications 1 à 10, où les au moins deux électrodes (70, 71) sont des circuits imprimés recouverts de cuivre.

12. L'unité de pompe de drainage selon une des revendications 1 à 11, où les au moins deux électrodes (70, 71) sont disposées sur le même circuit imprimé (72).

13. L'unité de pompe de drainage selon une des revendications 1 à 12, où les au moins deux électrodes (70, 71) sont disposées sur une paroi intérieure (13) du boîtier de pompe d'aspiration (1).

14. L'unité de pompe de drainage selon la revendication 13, où la paroi intérieure (13) du boîtier de pompe d'aspiration (1) présente un approfondissement pour la réception des au moins deux électrodes (70, 71), où l'approfondissement est calculé de sorte que les au moins deux électrodes (70, 71) de la paroi intérieure (13) ne font pas saillie en direction du côté intérieur du récipient.

15. Un capteur de niveau (7) pour l'utilisation dans une unité de pompe de drainage selon une des revendications 1 à 14, **caractérisé en ce que** le capteur de niveau (7) présente au moins deux électrodes (70, 71), lesquelles sont disposées l'une à côté de l'autre dans un plan commun, mais espacées l'une par rapport à l'autre, et lesquelles s'étendent sur une distance commune, qu'une première des au moins deux électrodes (70) est fabriquée d'une seule pièce et au moins une deuxième de ces électrodes (71) est formée de manière segmentée et que la première électrode (70) fabriquée d'une pièce est formée comme électrode d'émission et la deuxième électrode (71) est formée comme électrode de réception, où un signal d'excitation d'un modulateur (80) peut être appliquée à la première électrode (70) fabriquée d'une seule pièce.

16. Un capteur de niveau selon la revendication 15, où le signal d'excitation peut être couplé de manière capacitive dans la deuxième électrode (71) formée de manière segmentée.
